# EUROPEAN PATENT SPECIFICATION

(11) **EP 0 687 659 B1**
(45) Date of publication and mention of the grant of the patent: **06.05.1998**
(21) Application number: 94907671.5
(22) Date of filing: 23.02.1994
(51) Int. Cl.: C07C 19/08, C07C 17/00, C07C 17/26, B01J 23/42, B01J 23/44, B01J 23/46, B01J 27/125

(54) **PROCESS FOR PRODUCING 1,1,1,3,3-PENTAFLUOROPROPANE**
VERFAHREN ZUR HERSTELLUNG VON 1, 1, 1, 3, 3-PENTAFLUOROPROPAN
PROCEDE DE PRODUCTION DE 1,1,1,3,3-PENTAFLUOROPROPANE

(30) Priority: 05.03.1993 JP 70850/93
(43) Date of publication of application: 20.12.1995
(73) Proprietor: DAIKIN INDUSTRIES, LTD., Kita-ku, Osaka 530 (JP)
(72) Inventor: SEKI, Eiji Yodogawa Works of Daikin Industries Ltd, Settsu-shi, Osaka 566 (JP); AOYAMA, Hirokazu Yodogawa Works of, Settsu-shi Osaka 566 (JP)
(74) Representative: TER MEER STEINMEISTER & PARTNER GbR
(86) International application number: JP9400287
(87) International publication number: WO9420440

(56) References cited:
- EP-A- 0 611 744
- JP-A- 2 204 443
- JP-A- 3 052 828
- US-A- 2 942 036
- US-A- 5 157 171

## Description

This invention relates to a method of producing a useful compound of 1,1,1,3,3-pentafluoropropane that can be substituted for the CFC and HCFC having been used as refrigerants, blowing agents, and cleaning agents.

### Prior Art

As the production method of 1,1,1,3,3-pentafluoropropane, it is known that 2,2,3-trichloro-1,1,1,3,3-pentafluoropropane as a raw material is reduced with hydrogen by using a palladium catalyst (U.S. Patent 2942036). The method, however, is unsuitable as an industrial method for producing 1,1,1,3,3-pentafluoropropane because the yield of the product is low and a large amount of a by-product, 1,1,1,3,3-pentafluoropropene is formed.

### Objectives of the invention

This invention is designed to produce 1,1,1,3,3-pentafluoropropane with high yield.

### The constitution of the invention

As a result of closely studying the processes for producing 1,1,1,3,3-pentafluoropropane, the inventors found a process by which the targeted material can be obtained with a high yield when 2,2-dichloro-1,1,1,3,3-pentafluoropropane as a raw material is reduced with hydrogen in a vapor phase in the presence of a catalyst composed of at least one chosen from the group of palladium, platinum, and rhodium. The invention has thus been produced.

The point of this invention is thus a method of producing 1,1,1,3,3-pentafluoropropane with high yield, in which 2,2-dichloro-1,1,1,3,3-pentafluoropropane as a raw material is reduced with hydrogen, usually at a temperature from 30° to 450°C , in a vapor phase in the presence of a catalyst composed of at least one chosen from the group of palladium, platinum, and rhodium.

As a vapor-phase reaction, a fixed-bed reactor or a fluidized-bed reactor can be used.

A catalyst composed of at least one chosen from the group of palladium, platinum, and rhodium is preferable to be carried on a carrier composed of at least one carrier chosen from the group of active carbon, alumina, silica gel, titanium oxide, and zirconia.

Although having little influence on the reaction, the particle size of carriers is preferably from 0.1 to 100 mm.

As for catalyst concentration on the carriers, although a wide range from 0.05% to 10% can be used, a concentration of from 0.5% to 5% is usually recommended.

The reaction temperature is usually from 30° to 450°C , preferably from 70° to 400°C .

In the reduction reaction of 2,2-dichloro-1,1,1,3,3-pentafluoropropane with hydrogen, the ratio of hydrogen to the raw material can be changed widely. But hydrogenation is usually performed by using at least a stoichiometrical amount of hydrogen. To the total mole of starting material, for example, five or more moles of hydrogen can be used, which considerably exceed its stoichiometrical amount.

Reaction pressure is not particularly restricted. The reaction can be conducted under pressure, at a reduced pressure, or at normal pressure. It is preferable to conduct the reaction under pressure or at normal pressure because a more complicated apparatus is needed for reaction at reduced pressure.

The contact time is usually from 0.1 to 300 seconds, preferably from one to 30 seconds.

The raw material 2,2-dichloro-1,1,1,3,3-pentafluoropropane to be used in the reaction of this invention can be synthesized by a reaction of fluorodichloromethane (CHFCl₂) and tetrafluoroethylene (CF₂CF₂) at a temperature of between 0 ° and 150°C in the presence of a catalyst of aluminum chloride (AlCl₃) activated with trichlorofluoromethane (CCl₃F) (see U.S. Patent 5157171). In this reaction, 3,3-dichloro-1,1,1,2,2-pentafluoropropane and 1,3-dichloro-1,1,2,2,3-pentafluoropropane are simultaneously produced as by-products.

In addition, the raw material to be used in the reaction of this invention can also be obtained by a rearrangement reaction of 3,3-dichloro-1,1,1,2,2-pentafluoropropane and/or of 1,3-dichloro-1,1,2,2,3-pentafluoropropane as raw materials produced as by-products in the process by using an AlCl₃ catalyst that is activated with CCl₃F similarly as mentioned above, at a temperature of between 50° and 100°C .

The amount of AlCl₃ used as a catalyst in the reactions, including the rearrangement reaction, is from 0.1 to two equivalent, preferably from 0.3 to one equivalent. Inasmuch as the reaction pressure is not particularly restricted, the reaction can be conducted under pressure, at a reduced pressure, or at normal pressure. It is preferable, however, to conduct the reaction under pressure or at normal pressure because a more complicated apparatus will be needed for reaction at reduced pressure.

### The possibility of utilizing the invention in industry

According to this invention, the targeted material can be obtained with high yield when 2,2-dichloro-1,1,1,3,3-pentafluoropropane as a raw material is reduced with hydrogen in a vapor phase in the presence of a catalyst composed of at least one chosen from the group of palladium, platinum, and rhodium.

### Examples

This invention will be explained more concretely in the following examples.

### Example 1

Nineteen cc of platinum catalyst carried on active carbon (particle size 3 mm) at 0.5% concentration were infused into a reactor made of SUS-316 (2 cm inside diameter and 40 cm in length). The reactor was heated to 180°C using an electric furnace during exposure to nitrogen gas. After the desired temperature was attained, gaseous 2,2-dichloro-1,1,1,3,3-pentafluoropropane that had been gasified in advance was introduced into the reactor at 16.4 cc/min., together with hydrogen at 65.6 cc/min. The reaction temperature was kept at 180°C . The reaction pressure was 1.013 × 10⁵ Pa, while the contact time was 8.4 sec. The produced gas was washed with water and dried with calcium chloride. It was then analyzed by gas chromatography. The result is shown in Table 1.

### Example 2

In the same reactor as used in Example 1, 18 cc of palladium catalyst carried on active carbon (particle size 3 mm) at 0.5% concentration were packed. The reactor was heated to 180°C in an electric furnace during exposure to nitrogen gas. After the desired temperature was attained, gaseous 2,2-dichloro-1,1,1,3,3-pentafluoropropane that had been gasified in advance was introduced into the reactor at 24 cc/min., together with hydrogen at 96 cc/min. The reaction temperature was kept at 180°C . The reaction pressure was 1.013 × 10⁵ Pa and the contact time was 5.4 sec. The produced gas was analyzed by gas chromatography after washing with water and drying with calcium chloride. The result is shown in Table 1.

### Example 3

Infused into the same reactor as used in Example 1 were 18 cc of rhodium catalyst carried on active carbon (particle size 1 mm) at 0.5% concentration. The reactor was heated to 200 °C in an electric furnace during exposure to nitrogen gas. After the desired temperature was reached, gaseous 2,2-dichloro-1,1,1,3,3-pentafluoropropane that had been gasified in advance was introduced into the reactor at 24 cc/min., together with hydrogen at 96 cc/min. The reaction temperature was kept at 200°C . The reaction pressure was 1.013 × 10⁵ Pa and the contact time was 5.2 sec. The produced gas was analyzed by gas chromatography after washing with water and drying with calcium chloride. The result is shown in Table 1.

### Example 4

Ten cc of palladium catalyst carried on alumina (particle size 3 mm) at 0.5% concentration were packed into a reactor made of SUS 316 (2 cm inside diameter and 40 cm length). The reactor was heated to 190°C in an electric furnace during exposure to nitrogen gas. After the target temperature was reached, gaseous 2,2-dichloro-1,1,1,3,3-pentafluoropropane that had been gasified in advance was introduced into the reactor at 16.4 cc/min., together with hydrogen at 65.6 cc/min. The reaction temperature was kept at 190°C . The reaction pressure was 3.04 × 10⁵ Pa and the contact time was 12 sec. The produced gas was analyzed by gas chromatography after washing with water and drying with calcium chloride. The result is shown in Table 1.

**Table 1**

| Example | Conversion (%) | Selectivity (%) |
|---|---|---|
| 1 | 99.7 | 98.5 |
| 2 | 99.8 | 99.0 |
| 3 | 99.5 | 97.8 |
| 4 | 99.7 | 98.2 |

## Claims

1. A method of producing 1,1,1,3,3-pentafluoropropane characterised by reducing 2,2-dichloro-1,1,1,3,3-pentafluoropropane by making it react with hydrogen in a vapor phase in the presence of a catalyst composed of at least one chosen from the group of palladium, platinum, and rhodium.

2. A production method as defined in Claim 1, in which the catalyst is carried on a carrier composed of at least one chosen from the group of active carbon, alumina, silica gel, titanium oxide, and zirconia.

3. A production method as defined in Claim 2, in which the catalyst's concentration on each carrier is from 0.05% to 10%.

4. A production method as defined in any of Claims 1 through 3, in which carrier particle size ranges from 0.1 to 100 mm.

5. A production method as defined in any of Claims 1 through 4, in which hydrogenation is conducted by using at least a stoichiometrical amount of hydrogen to 2,2-dichloro-1,1,1,3,3-pentafluoropropane.

6. A production method as defined in any of Claims 1 through 5, in which the reaction is conducted at a temperature of between 30° and 450°C .

7. A production method as defined in any of Claims 1 through 6, in which the reaction is a vapor-phase reaction in a fixed-bed reactor or in a fluidized-bed reactor.

8. A production method as defined in any of Claims 1 through 7, in which the reaction is performed under pressure or at normal pressure.

9. A production method as defined in any of Claims 1 through 8, in which the reaction has a contact time with a catalyst of between 0.1 and 300 sec.

10. A production method as defined in any of Claims 1 through 9, in which 2,2-dichloro-1,1,1,3,3-pentafluoropropane is synthesized from fluorodichloromethane and tetrafluoroethylene in the presence of a catalyst.

11. A production method as defined in Claim 10, in which 2,2-dichloro-1,1,1,3,3-pentafluoropropane is synthesized from fluorodichloromethane and tetrafluoroethylene at a temperature of between 0° and 150°C in the presence of an aluminum chloride catalyst activated with trichlorofluoromethane.

12. A production method as defined in any of Claims 1 through 9, in which 2,2-dichloro-1,1,1,3,3-pentafluoropropane is synthesized by a rearrangement reaction of 3,3-dichloro-1,1,1,2,2-pentafluoropropane and/or 1,3-dichloro-1,1,2,2,3-pentafluoropropane, which are produced as by-products in the processes as defined in Claims 10 or 11, in the presence of a catalyst.

13. A production method as defined in Claim 12, in which the rearrangement reaction is performed at a temperature of between 50° and 100°C by using a catalyst of aluminum chloride activated with trichlorofluoromethane.

14. A production method as defined in any of Claims 10 through 13, in which a catalyst is used in an amount of from 0.1 to 2 equivalent.

15. A production method as defined in any of Claims 10 through 14, in which the synthetic reaction is performed under pressure or at normal pressure.

## Patentansprüche

1. Verfahren zur Herstellung von 1,1,1,3,3-Pentafluorpropan, gekennzeichnet durch die Reduzierung von 2,2-Dichlor-1,1,1,3,3-pentafluorpropan, in dem dieses mit Wasserstoff in einer Dampfphase in Gegenwart eines Katalysators, der aus mindestens einem, gewählt aus der Gruppe Palladium, Platin und Rhodium, zusammengesetzt ist, umgesetzt wird.

2. Herstellungsverfahren nach Anspruch 1, wobei der Katalysator auf einem Träger getragen wird, der aus mindestens einem, gewählt aus der Gruppe Aktivkohle, Aluminiumoxid, Silicagel, Titanoxid und Zirconoxid, zusammengesetzt ist.

3. Herstellungsverfahren nach Anspruch 2, wobei die Konzentration des Katalysators auf dem jeweiligen Träger 0,05 bis 10 % beträgt.

4. Herstellungsverfahren nach mindestens einem der Ansprüche 1 bis 3, wobei die Teilchengröße des Trägers im Bereich von 0,1 bis 100 mm liegt.

5. Herstellungsverfahren nach mindestens einem der Ansprüche 1 bis 4, wobei die Hydrierung durch Verwendung mindestens einer stöchiometrischen Menge an Wasserstoff gegenüber 2,2-Dichlor-1,1,1,3,3-pentafluorpropan durchgeführt wird.

6. Herstellungsverfahren nach mindestens einem der Ansprüche 1 bis 5, wobei die Umsetzung bei einer Temperatur zwischen 30 und 450°C durchgeführt wird.

7. Herstellungsverfahren nach mindestens einem der Ansprüche 1 bis 6, wobei die Umsetzung eine Dampfphasenumsetzung in einem Festbettreaktor oder in einem Wirbelbettreaktor ist.

8. Herstellungsverfahren nach mindestens einem der Ansprüche 1 bis 7, wobei die Umsetzung unter Druck oder bei Normaldruck durchgeführt wird.

9. Herstellungsverfahren nach mindestens einem der Ansprüche 1 bis 8, wobei die Umsetzung bei einer Kontaktzeit mit dem Katalysator zwischen 0,1 und 300 s durchgeführt wird.

10. Herstellungsverfahren nach mindestens einem der Ansprüche 1 bis 9, wobei 2,2-Dichlor-1,1,1,3,3-pentafluorpropan aus Fluordichlormethan und Tetrafluorethylen in Gegenwart eines Katalysators hergestellt wird.

11. Herstellungsverfahren nach Anspruch 10, wobei 2,2-Dichlor-1,1,1,3,3-pentafluorpropan aus Fluordichlormethan und Tetrafluorethylen bei einer Temperatur zwischen 0° und 150°C in Gegenwart eines mit Trichlorfluormethan aktivierten Aluminiumchlorid-Katalysators hergestellt wird.

12. Herstellungsverfahren nach mindestens einem der Ansprüche 1 bis 9, wobei 2,2-Dichlor-1,1,1,3,3-pentafluorpropan durch eine Umlagerungsreaktion von 3,3-Dichlor-1,1,1,2,2-pentafluorpropan und/oder 1,3-Dichlor-1,1,2,2,3-pentafluorpropan, welche als Nebenprodukte bei den in den Ansprüchen 10 oder 11 definierten Verfahren erzeugt werden, in Gegenwart eines Katalysators hergestellt wird.

13. Herstellungsverfahren nach Anspruch 12, wobei die Umlagerungsreaktion bei einer Temperatur zwischen 50 und 100°C durch Verwendung eines Katalysators aus mit Trichlorfluormethan aktiviertem Aluminiumchlorid durchgeführt wird.

14. Herstellungsverfahren nach mindestens einem der Ansprüche 10 bis 13, wobei der Katalysator in einer Menge von 0,1 bis 2 Äquivalenten verwendet wird.

15. Herstellungsverfahren nach mindestens einem der Ansprüche 10 bis 14, wobei die Synthesereaktion unter Druck oder bei Normaldruck durchgeführt wird.

## Revendications

1. Procédé pour la production de 1,1,1,3,3-pentafluoropropane, caractérisé en ce que l'on réduit du 2,2-dichloro-1,1,1,3,3-pentafluoropropane en le faisant réagir avec de l'hydrogène en phase gazeuse en présence d'un catalyseur composé d'au moins un élément choisi dans le groupe du palladium, du platine et du rhodium.

2. Procédé de production selon la revendication 1, dans lequel le catalyseur est supporté par un support composé d'au moins un constituant choisi dans le groupe du charbon actif, de l'alumine, du silica gel, de l'oxyde de titane et du zirconium.

3. Procédé de production selon la revendication 2, dans lequel la concentration en catalyseur sur le support varie de 0,05 à 10%.

4. Procédé de production selon l'une quelconque des revendications 1 à 3, dans lequel la taille particulaire du support varie entre 0,1 et 100 mm.

5. Procédé de production selon l'une quelconque des revendications 1 à 4, dans lequel l'hydrogénation est réalisée par utilisation d'au moins une quantité stoechiométrique d'hydrogène par rapport au 2,2-dichloro-1,1,1,3,3-pentafluoropropane.

6. Procédé de production selon l'une quelconque des revendications 1 à 5, dans lequel la réaction est réalisée à une température comprise entre 30 et 450°C.

7. Procédé de production selon l'une quelconque des revendications 1 à 6, dans lequel la réaction est une réaction en phase gazeuse dans un réacteur à lit fixe ou dans un réacteur à lit fluidisé.

8. Procédé de production selon l'une quelconque des revendications 1 à 7, dans lequel la réaction est réalisée sous pression ou à une pression normale.

9. Procédé de production selon l'une quelconque des revendications 1 à 8, dans lequel le temps de contact avec le catalyseur pendant la réaction varie entre 0,1 et 300 s.

10. Procédé de production selon l'une quelconque des revendications 1 à 9, dans lequel le 2,2-dichloro-1,1,1,3,3-pentafluoropropane est synthétisé à partir de fluorodichlorométhane et de tétrafluoroéthylène en présence d'un catalyseur.

11. Procédé de production selon la revendication 10, dans lequel le 2,2-dichloro-1,1,1,3,3-pentafluoropentane est synthétisé à partir de fluorodichlorométhane et de tétrafluoroéthylène à une température comprise entre 0 et 150°C en présence d'un catalyseur à base de chlorure d'aluminium activé avec du trichlorofluorométhane.

12. Procédé de production selon l'une quelconque des revendications 1 à 9, dans lequel le 2,2-dichloro-1,1,1,3,3-pentafluoropropane est synthétisé par réarrangement du 3,3-dichloro-1,1,1,2,2-pentafluoropropane et/ou du 1,3-dichloro-1,1,2,2,3-pentafluoropropane, lesquels sont formés en tant que sous produits dans les procédés tels que définis aux revendications 10 ou 11 en présence d'un catalyseur.

13. Procédé de production selon la revendication 12, dans lequel le réarrangement est réalisé à une température comprise entre 50 et 100°C par utilisation d'un catalyseur à base de chlorure d'aluminium activé avec du trichlorofluorométhane.

14. Procédé de production selon l'une quelconque des revendications 10 à 13, dans lequel un catalyseur est utilisé dans une quantité comprise entre 0,1 et 2 équivalents.

15. Procédé de production selon l'une quelconque des revendications 10 à 14, dans lequel la réaction de synthèse est réalisée sous pression ou à une pression normale.
